# EUROPEAN PATENT SPECIFICATION

(11) **EP 2 895 084 B1**
(45) Date of publication and mention of the grant of the patent: **28.10.2020**
(21) Application number: 13774269.8
(22) Date of filing: 10.09.2013
(51) Int. Cl.: A61B 17/16, A61B 17/17, A61B 17/34

(54) **MULTIHOLE DRILL SLEEVE WITH PROTECTION SLEEVE**
MEHRLOCH-BOHRHÜLSE MIT SCHUTZHÜLSE
MANCHON DE FORET MULTITROU POURVU D'UN MANCHON DE PROTECTION

(30) Priority: 14.09.2012 US 201261701070 P
(43) Date of publication of application: 22.07.2015
(60) Divisional application: 16199392.8
(73) Proprietor: Synthes GmbH, 4436 Oberdorf (CH)
(72) Inventor: FELDER, Martin, 4500 Solothurn (CH)
(74) Representative: Klunker IP Patentanwälte PartG mbB
(86) International application number: PCT/US2013/058960
(87) International publication number: WO 2014/043093

(56) References cited:
- EP-A1- 0 648 470
- WO-A2-2005/037065
- DE-C1- 3 412 362
- US-A- 5 766 221
- US-A1- 2002 077 530

## Description

### Background Information

Systems and methods for bone fixation sometimes require the insertion of a bone fixation device to, for example, secure fragments of a bone in a corrected alignment and/or to increase a strength of the bone. For example, a bone fixation device may be inserted into a medullary cavity of a bone and aligned in a target portion of the medullary cavity via a guide wire marking a prescribed path for insertion of the bone fixation device. Proper positioning of the guide wire is very important since even a minor misalignment may lead to the bone fixation element following an incorrect path through the bone. In the worst cases, this may cause the bone fixation element to extend laterally out of a shaft of the bone. In many other cases, misalignment may lead to insertion of the bone fixation device into the medullary cavity in a laterally offset position which may lead to further fracture, prevent reaming of the medullary cavity and/or may inhibit insertion of the bone fixation device to the desired depth in the bone.

WO 2005/037065 A2 discloses a surgical drill guide. The drill guide may include a first drill guide body having a distal end for placement substantially adjacent a bone, a second drill guide body slidable with respect to the first drill guide body and having a proximal end for contacting a portion of a drill bit, a handle associated with at least one of the first drill guide body and the second drill guide body, and a detent mechanism for releasably retaining the position of the first drill guide body with respect to the second drill guide body in predefined increments. The drill guide may include a locking member movable between a first position where it substantially prevents sliding of the second drill guide body with respect to the first drill guide body, and a second position where it permits sliding of the second drill guide body with respect to the first drill guide body. The locking member may be resiliently biased to the first position.

### Summary of the Invention

The present invention is directed to an aiming device for bone fixation, comprising a handle extending from a first end to a second end along a longitudinal axis and a first guide arm connected to the second end of the handle and having a first opening extending therethrough, the first guide arm being connected to a protection sleeve in combination with a second guide arm connected to the first guide arm and having a second opening extending therethrough and a drill guide configured and dimensioned for insertion through the second opening of the second guide arm, the drill guide having a first guide channel extending therethrough to guide an insertion of a gu ide wire into a bone, wherein the second guide arm is movable between a first configuration wherein the drill guide is axially movable relative to the second guide arm to a second configuration wherein the drill guide is not axially movable relative to the second guide arm.

### Brief Description of the Drawings

Several embodiments of the invention will be described in the following by way of example and with reference to the accompanying drawings in which:
Fig. 1 illustrates a first perspective of a bone fixation device according to an exemplary embodiment of the invention;
Fig. 2 illustrates a first partial cross-sectional view of the bone fixation device of Fig. 1;
Fig. 3 illustrates a second partial cross-sectional view of the bone fixation device of Fig. 1;
Fig. 4 illustrates a second perspective view of the bone fixation device of Fig. 1;
Fig. 5 illustrates a perspective view of an extension arm of the device of Fig. 1;
Fig. 6 illustrates a partial cross-sectional view of extension of Fig. 5;
Fig. 7 illustrates a perspective view of a housing of the device of Fig. 1;
Fig. 8 illustrates a partial cross-sectional view of the housing of Fig. 8;
Fig. 9 illustrates a first perspective view of a blocking element of the device of Fig. 1;
Fig. 10 illustrates a second perspective view of the blocking element of Fig. 9;
Fig. 11 illustrates a third perspective view of the blocking element of Fig. 9;
Fig. 12 illustrates a partial cross-sectional side view of the blocking element of Fig. 9;
Fig. 13 illustrates a perspective view of a protection sleeve of the device of Fig. 1;
Fig. 14 illustrates a cross-sectional view of the protection sleeve of Fig. 13;
Fig. 15 illustrates a first perspective view of a drill guide of the device of Fig. 1;
Fig. 16 illustrates a second perspective view of the drill guide of Fig. 15;
Fig. 17 illustrates a partial cross-sectional view of the drill guide of Fig. 15;
Fig. 18 illustrates a third perspective view of the drill guide of Fig. 15;
Fig. 19 illustrates a device according to an alternative embodiment in a first operative configuration;
Fig. 20 illustrates the device of Fig. 19 in a second operative configuration;
Fig. 21 illustrates a device according to an alternate embodiment of the invention;
Fig. 22 illustrates the device of Fig. 21 in a first operative configuration;
Fig. 23 illustrates the device of Fig. 21 in a second operative configuration;
Fig. 24 illustrates a first perspective view of a device of Fig. 21 in operation;
Fig. 25 illustrates a second perspective view of a device of Fig. 21 in operation;
Fig. 26 illustrates a third perspective view of a device of Fig. 21 in operation;
Fig. 27 illustrates a first perspective view of a device according to another illustrative example;
Fig. 28 illustrates a partial see-through view of the device of Fig. 27;
Fig. 29 illustrates a partial cross-sectional view of the device of Fig. 27 in an axially and rotationally locked position;
Fig. 30 illustrates a partial cross-sectional view of the device of Fig. 27 in an unlocked position;
Fig. 31 illustrates a partial see-through view of the device of Fig. 27 in the axially and rotationally locked position;
Fig. 32 illustrates a partial see-through view of the device of Fig. 27 in an unlocked position;
Fig. 33 illustrates the device of Fig. 27 in a first operative configuration;
Fig. 34 illustrates the device of Fig. 27 in a second operative configuration;
Fig. 35 illustrates the device of Fig. 27 in a third operative configuration;
Fig. 36 illustrates the device of Fig. 27 in a fourth operative configuration; and
Fig. 37 illustrates the device of Fig. 27 in a fifth operative configuration.

### Detailed Description

The present invention is directed to an aiming device configured to aid in an insertion of a guide wire into a bone (e.g., into a medullary cavity) in a target position (e.g., in alignment with the medullary cavity). Specifically, the aiming device according to the invention is formed as a multi-hole trocar configured for removable attachment to a protection sleeve and movable relative thereto from an insertion configuration with a pointed distal tip of the trocar extending distally beyond a distal end of the protection sleeve and an operative configuration in which the protection sleeve is moved distally relative to the distal tip of the trocar, as will be described in greater detail later on. The trocar further comprises a drill guide including a plurality of channels extending longitudinally therethrough, each of the channels being configured to receive a guide wire. The drill guide may be rotated relative to the protection sleeve to alter the positions of the channels to obtain an optimum position of a selected one of the channels so that a guide wire inserted therethrough will pass into the bone along a desired path. Once this desired position has been achieved, a locking mechanism is activated to prevent the drill guide from rotating away from the desired position and to prevent axial movement thereof relative to the trocar. Where conventional devices are often required to be removed from the body to reposition a drill guide, an exemplary device according to the invention permits rotation and axial movement of the drill guide after the device has been inserted over a target portion of a bone while the device remains in place within the body. The exemplary device according to the invention may be used to guide a guide wire or other device into any bone of the body. It is noted that although the exemplary embodiment is disclosed with respect to a device configured for insertion into a medullary canal of a long bone, the exemplary system according to the invention may be employed in any other bone without deviating from the scope of the invention. As used herein, the term "proximal" refers to a direction approaching a physician or other user and the term "distal" refers to a direction approaching a target treatment site in the body.

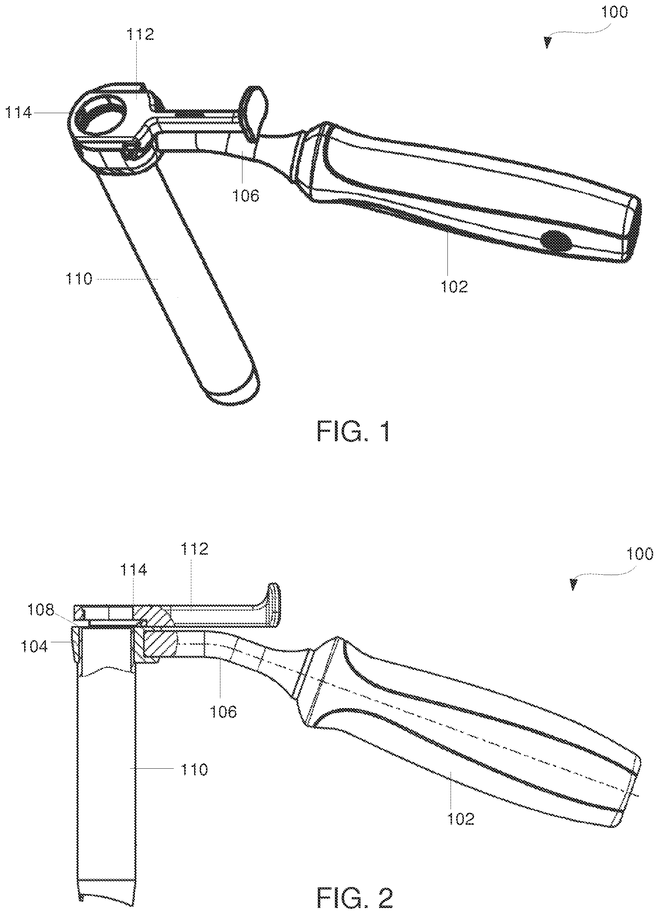

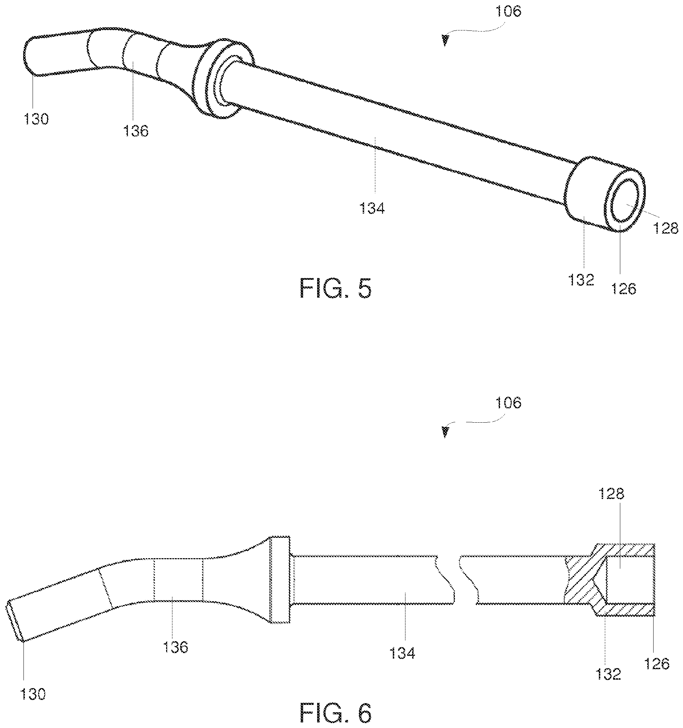

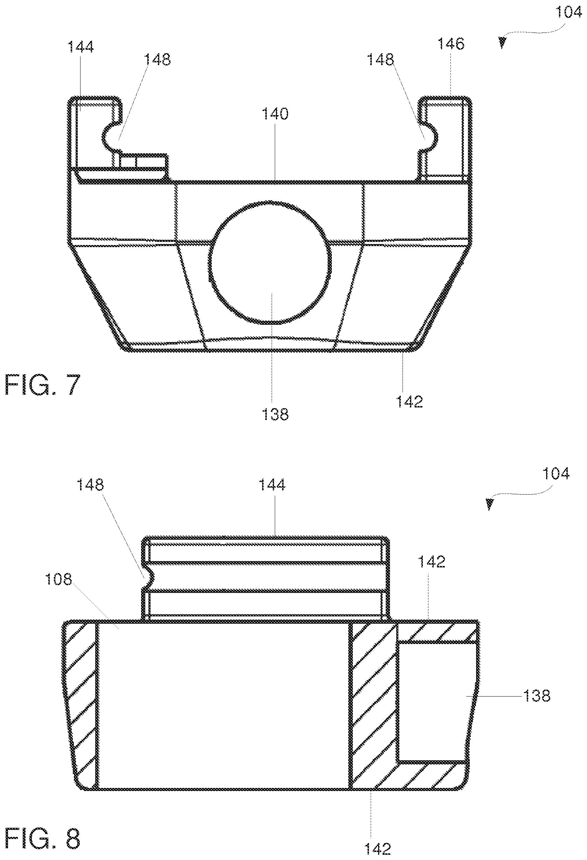

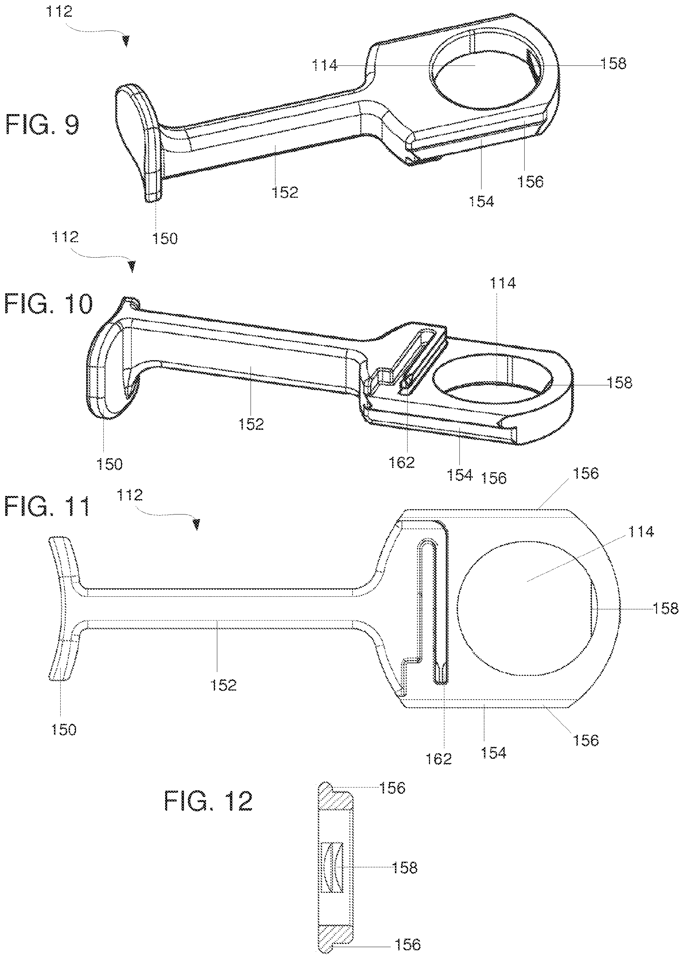

Figs. 1 - 20 depict an exemplary device 100 according to the present invention. The device comprises a handle 102 operably connected to a housing 104 via an extension arm forming an extension 106, the housing 104 and the extension 106 forming a first guide arm. The housing 104 comprises an opening 108 extending therethrough configured and dimensioned to receive a protection sleeve 110 therein. In an exemplary embodiment, the protection sleeve 110 is permanently attached to the housing 104 through, for example, welding. The housing 104 is also permanently attached to the extension arm 106 and handle 102. A blocking element 112 forming a second guide arm is provided on a proximal face of the extension 106, the blocking element 112 having an opening 114 extending therethrough to permit insertion of a drill guide formed by a drill sleeve 116 therethrough. The blocking element 112 is removable from the housing 104 for cleaning. The drill sleeve 116 is configured to extend through the protection sleeve 110 and distally beyond a distal end thereof, as will be described in greater detail later on. A proximal end of the drill sleeve 116 comprises a locking mechanism configured to engage the opening 114 (e.g., with a threaded or ratcheted engagement), as will be described in greater detail later on. In a first operative configuration, the locking mechanism is disengaged from the opening 114 to permit longitudinal movement and rotation of the drill sleeve 116 relative to the opening 114 of the blocking element 112. In a second operative configuration, the locking mechanism engages the opening 114 preventing axial movement of the drill sleeve 116 relative to the blocking element 112 while still permitting rotation thereof relative to the blocking element 112, as will be described in greater detail with respect to an exemplary method. The locking mechanism remains in the second operative configuration until a user applies a force to the blocking element 112 to cause a movement thereof to the first configuration. The exemplary device according to the invention allows a physician or other user to rotate the drill sleeve 116 to a desired orientation without having to apply a constant distal force to hole the drill sleeve 116 against the bone, as will also be described in greater detail later on.

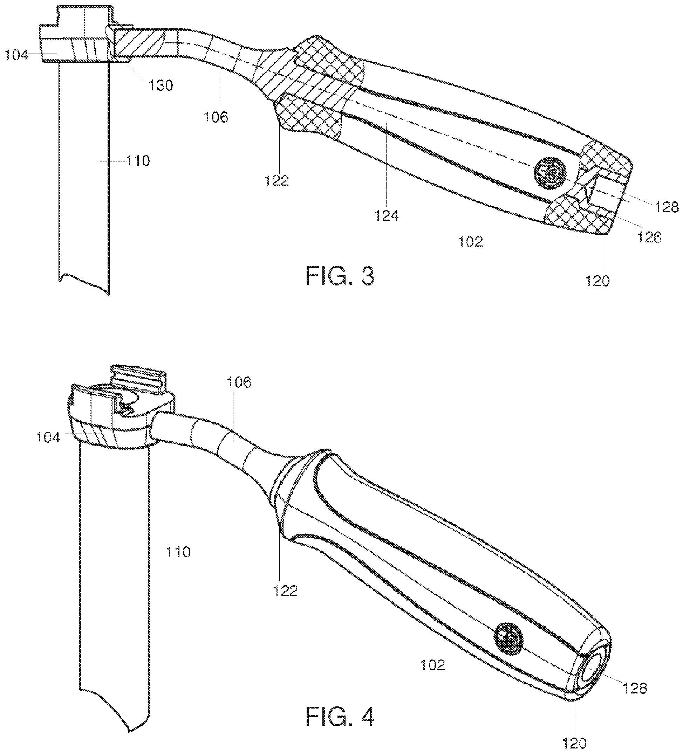

Figs. 3 - 8 depict a detailed view of the exemplary handle 102, extension 106 and housing 104 according to the invention. The handle 102 extends from a first end 120 to a second end 122 and comprises an elongated channel 124 extending longitudinally therethrough. The channel 124 is configured to receive a first portion of the extension 106 therein. The handle 102 may be formed in any ergonomic configuration, as those skilled in the art will understand by, for example, applying a silicone overmold over an outer wall of the handle 102. The extension 106 extends from a first end 126 having an opening 128 to a second end 130 configured to engage with the housing 104. The opening 128 is configured to hold the extension 106 within a mold during silicone injection, as those skilled in the art will understand. The first end 126 comprises an enlarged diameter portion 132 extending the length of the opening 128, the enlarged diameter portion 132 providing an added strength to the location of the opening 128, as those skilled in the art will understand. An elongated shaft 134 extends away from the enlarged diameter portion 132 to the second end 130 of the head 102. In an exemplary embodiment, the shaft 134 has a substantially circular cross-sectional shape although any other shape may be employed without deviating from the scope of the invention. The extension 106 further comprises a tapered portion 136 extending out of the head 102, the tapered portion 136 angled so that when the handle 102 is positioned at a desired angle relative to the bone (not shown), the housing 104 attached to the second end 130 lies in a plane extending substantially orthogonal to a longitudinal axis of the bone, as will be described in greater detail later on. The second end 130 is permanently attached to the housing 104 via, for example, laser welding. It is noted, however, that any other attachment mechanism may be used without deviating from the scope of the invention.

The housing 104 is formed with a substantially planar body having an opening 138 extending laterally thereinto to receive the second end 130 of the extension 106. The opening 108 extends substantially perpendicularly through the housing 104 from a proximal face 140 to a distal face 142. A pair of grooved arms 144, 146 is provided on the proximal face 142, with each of the grooved arms 144, 146 having a slot 148 formed therein substantially parallel to a plane of the housing 104 so that the blocking element 112 is slidably receivable therein in parallel alignment to the housing 104. In an exemplary embodiment, the arms 144, 146 are positioned on opposing ends of the proximal face 140 so that the blocking element 112 may be inserted from a direction of the handle 102 and extension 106, as shown in Fig. 2.

The blocking element 112 extends from a first end having a tab 150 and along a lateral arm 152 to a substantially planar body portion 154 configured for placement adjacent the housing 104. Opposing side walls of the blocking element 112 comprise abutments 156 extending thereoutof and configured to engage the slots 148 of the housing 104. In an operative configuration, a physician or other user slides the abutments 156 along the slots 148 until the opening 114 is provisionally aligned with the opening 108. A spring rail 162 provided on the lower surface of the blocking element 112 prevents the blocking element 112 from sliding further than an alignment configuration with the housing 104. Specifically, the spring rail 162 engages the drill sleeve 116 inserted through the blocking element 112 and housing 104 to prevent further slidable movement thereof relative to one another.

The opening 114 of the blocking element also comprises a protrusion 158 extending out of a side wall thereof and into the channel by a predetermined distance. In an exemplary embodiment, the protrusion 158 is provided on a side wall located opposite the lateral arm 152. The protrusion 158 has a substantially planar wall 160 configured to interface with the drill sleeve 116 inserted therethrough. Specifically, a width of the opening 114 at the protrusion 158 is sufficient to permit insertion of the drill sleeve 116 therethrough. As will be described in greater detail below, the blocking element 112 is movable relative to the housing 104 so that, in a first position, the protrusion 158 frictionally engages the drill sleeve 116 to prevent axial movement thereof and, in a second position, the protrusion 158 does not contact the drill sleeve 116. Specifically, in the first position, the protrusion 158 frictionally engages one of the first and second threaded portions 184, 188.

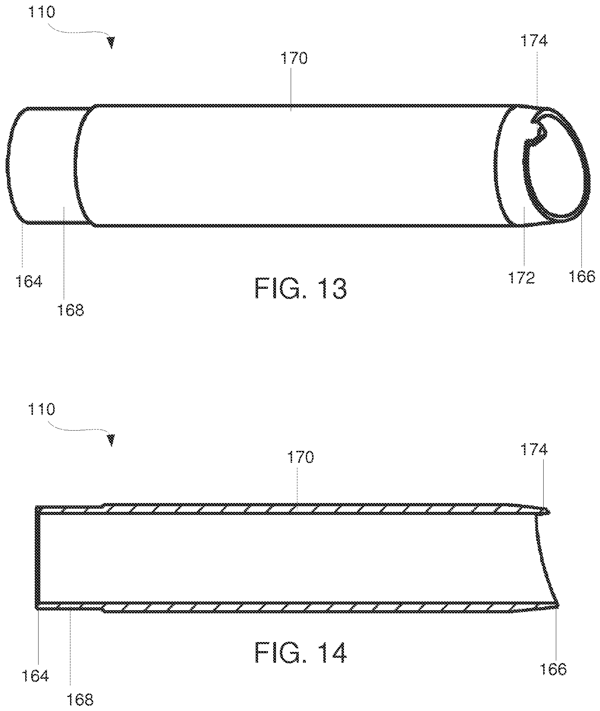

Figs. 13 - 14 depict an exemplary protection sleeve 110 according to the invention. The protection sleeve 110 extends along a central longitudinal axis from a proximal end 164 to a distal end 166 and has a substantially circular cross-sectional shape. In an exemplary embodiment, a proximal portion 168 of the protection sleeve 110 has a reduced diameter selected to engage the opening 108. A distal portion 170 of the protection sleeve 110 has an increased diameter selected to prevent insertion thereof into the opening 108, thus preventing the protection sleeve 110 from being retracted proximally away from the bone in an operative configuration. The distal end 166 is formed with an angled wall configured to conform to an angle of a head of a target bone. The distal end 166 may also comprise a tapered portion 172 having a taper angle of approximately 45E, although any other angle may be used without deviating from the scope of the invention. As those skilled in the art will understand, the tapered portion 172 aids in insertion of the protection sleeve 110 to a target portion adjacent the bone. A pointed tip 174 provided on the distal end 166 is configured to grippingly engage the bone to lock a position of the protection sleeve 110 thereagainst.

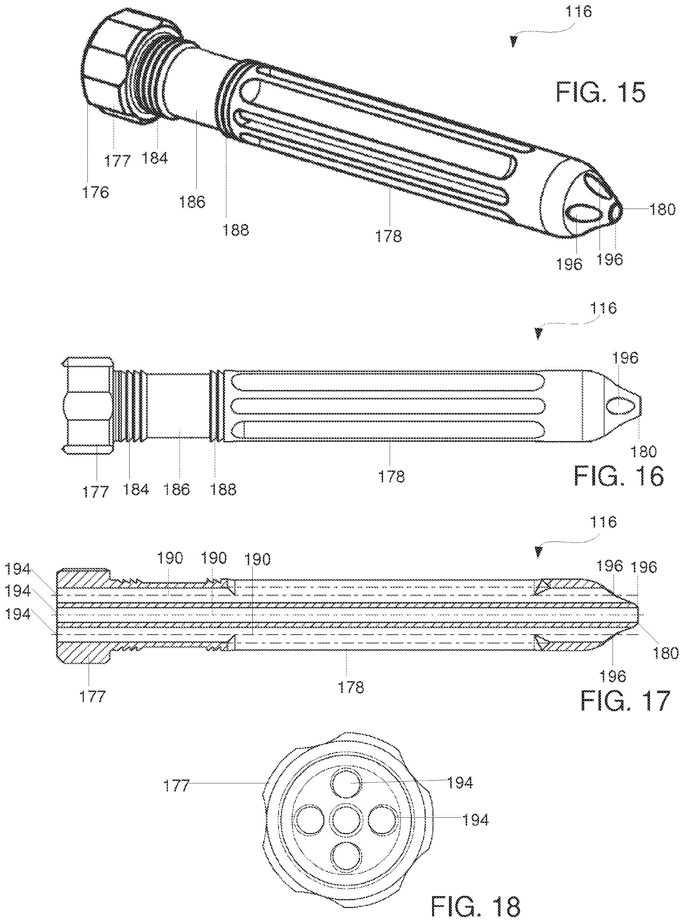

Figs. 15 - 18 depict an exemplary drill sleeve 116 according to the invention. The drill sleeve 116 extends from a proximal end 176 comprising a head 177 to a distal end 180 comprising a shaft 178 having a longitudinal axis. An outer diameter of the head 177 is greater than a diameter of the opening 114 of the blocking element to prevent axial movement of the head 177 distally into the blocking element 112. A first threaded portion 184 may extend distally from the head 177 and may be configured to threadedly engage a threaded portion of the opening 114. A non-threaded portion 186 extends distally from the first threaded portion 184. A second threaded portion 188 may be provided distally of the non-threaded portion 186 and may be configured to threadedly engage the opening 114 in an axially locked configuration, as will be described in greater detail later on. A distal portion of the shaft 178 is provided with a taper configured to aid in insertion thereof to the target location in the body, as those skilled in the art will understand. The drill sleeve 116 comprises a plurality of elongated channels 190 extending longitudinally therethrough and open at openings 194 at the proximal end 176 and openings 196 at the distal end 180. The channels 190 may be provided in any configuration through the drill sleeve 116. Additionally, any number of channels 190 may be used without deviating from the scope of the invention. In one exemplary embodiment, a first channel 190 may extend through a central longitudinal axis of the drill sleeve 116. Four additional channels 190 may be provided around the first channel and may be separated from one another by approximately 90°.

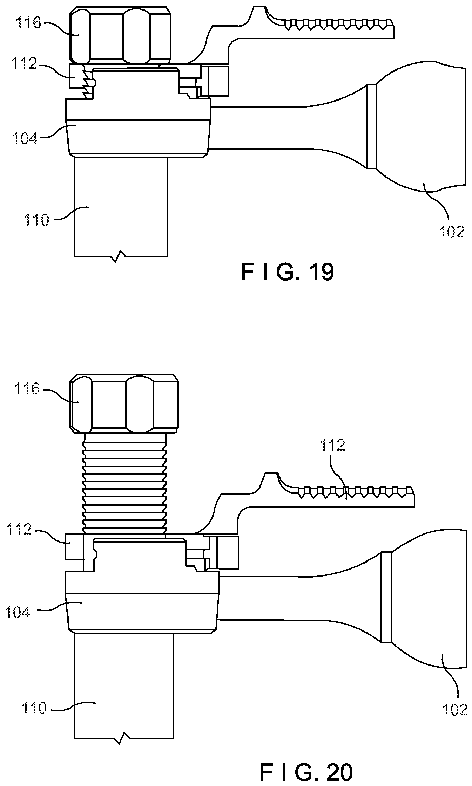

As shown in Figs. 19 - 20, in an exemplary method, a physician or other user forms an incision at a target entry point adjacent to, for example, a head of a target bone. The proximal portion 168 of the protection sleeve 110 is then positioned in the opening 108 of the housing. The drill sleeve 116 is then inserted through the opening 114 and opening 108. The distal end 166 is then inserted into the body until contact is made with the head of the bone. The physician then applies distally directed pressure to the device 100 to maintain a position of the protection sleeve 110 over the bone. In a first insertion configuration, the physician moves the blocking element 112 medially toward the body to release an axial locking force on the drill sleeve 116. In this configuration, the protrusion 158 is moved away from the drill sleeve 116 and out of alignment with the opening 108. In this configuration, the drill sleeve 116 is permitted to move axially relative to the device 100 to aid in proper positioning thereof. Once the distal end 180 of the drill sleeve 116 is in a desired position in contact with the bone, the physician moves the blocking element 112 to a blocked configuration by moving the blocking element laterally away from the body. In the blocked configuration, the protrusion 158 moves into the channel axis of the opening 108 to apply a radially compressive pressure to the drill sleeve 116 sufficient to prevent axial movement thereof. The pressure applied by the protrusion 158 may be selected to still permit rotation of the drill sleeve 116 about its longitudinal axis 182, thus permitting a physician to rotate the channels 190 into a desired position over the bone. The blocking element 112 may be moved from the insertion configuration to the blocking configuration as many times as may be needed to properly position the channels 190 over the bone. The exemplary device according to the invention is configured so that the physician does not need to apply constant pressure to the drill sleeve 116 to maintain its position in the bone. Additionally, the ergonomic design of the invention is easier to handle, thus reducing the time needed to perform a guide wire insertion procedure and reducing radiation exposure, as those skilled in the art will understand. After proper positioning of the drill sleeve 116, the physician may insert a guide wire (not shown) into a selected one of the channels 190 and perform the remainder of the bone fixation procedure according to systems and methods known in the art.

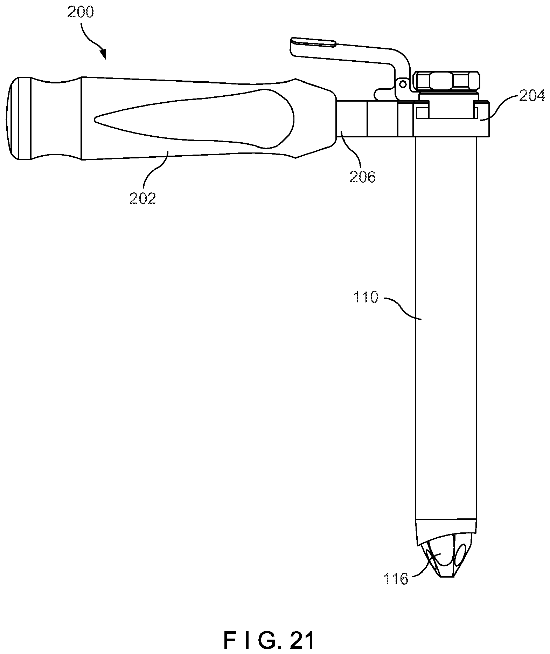

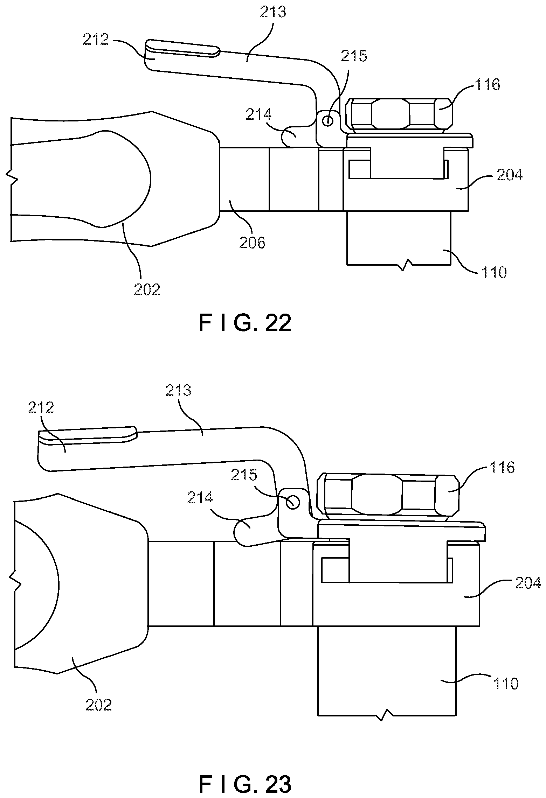

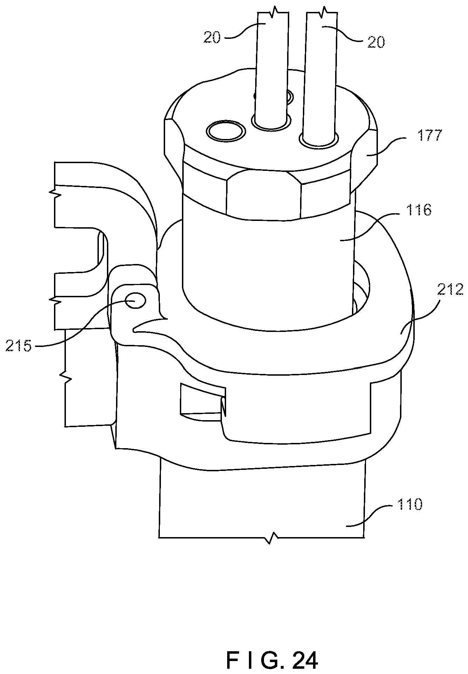

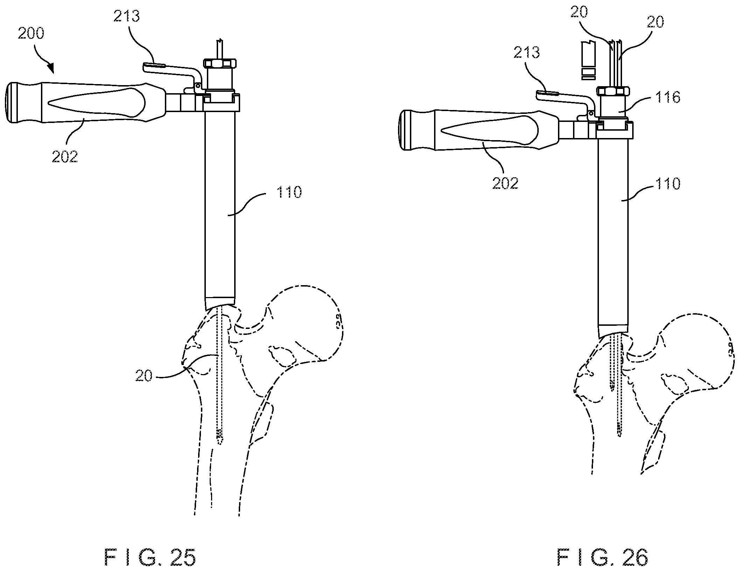

As shown in Figs. 21 - 26, a device 200 according to another embodiment of the invention is substantially similar to the device 100 except as pointed out below, with like elements referenced with like reference numerals. The device 200 comprises a handle 202 operably connected to a housing 204 via an extension 206. In an exemplary embodiment, the extension 206 may extend in longitudinal alignment with a longitudinal axis of the handle 202, although any other configuration may also be used without deviating from the scope of the invention. The housing 204 is formed substantially similarly to the housing 104 and comprises the opening 108 extending therethrough to receive the protection sleeve 110 therethrough. A blocking element 212 of the device 200 comprises the opening 114 configured to permit insertion of the drill sleeve 116 therethrough. The blocking element 212 also comprises a lever 213 connected to a base portion 214. The lever 213 is rotatably connected to the housing 204 via, for example, a pivot pin 215. As will be described in greater detail later on, the lever 213 may be pivotable relative to the housing from a first operative configuration as shown in Fig. 22 to a second operative configuration as shown in Fig. 23. In a first configuration, the lever 213 is positioned so that the base 214 rests extends substantially parallel to a longitudinal axis of the handle 202, as will be described hereinafter.

In accordance with an exemplary method, the device 200 is inserted into the body in an insertion configuration where the head 177 of the drill sleeve 116 is seated against the blocking element 212, as shown in Fig. 21. During insertion, a physician or other user applies a distally directed force to the lever 213, the force causing the lever 213 to pivot as shown in Fig. 23. As those skilled in the art will understand, engagement of the base 214 with the extension 206 limits pivotal movement of the lever 213. Pivoting the lever 213 causes the opening 114 of the blocking element 212 to move out of longitudinal alignment with the opening 108 of the housing 204. As with the device 100, this configuration causes the drill sleeve 116 to become frictionally locked and prevented from rotation or axial movement. Once the distal end 180 of the drill sleeve 116 has made contact with an outer surface of a target bone, the physician releases the lever 213 and permits the lever 213 to return to the biased configuration of Fig. 22 wherein the opening 114 of the blocking element 212 returns to longitudinal alignment with the opening 108 of the housing 204. In this configuration, the drill sleeve 116 is able to both move axially and rotationally relative to the protection sleeve 110. Thus, release of the lever 213 causes the protection sleeve to move distally toward the bone so that the distal end 166 of the protection sleeve 110 is in contact with the outer surface of the bone. In this configuration, both the drill sleeve 116 and the protection sleeve 110 are in direct contact with the target portion of the bone. The physician may then optionally rotate the drill sleeve 116 relative to the protection sleeve 110 to provisionally align the channels to a target insertion orientation. A guide wire 20 may then be inserted into a selected channel 190 of the drill sleeve 116. If the physician determines that a position of the guide wire 20 is not accurate, an additional guide wire 20 may be inserted through another one of the channels 190 without removing the device 200 and reinserting it into the body, as is generally done in conventional devices. If the physician determined that a position of the additional guide wire 20 is correct, the first guide wire 20 may be removed from the body.

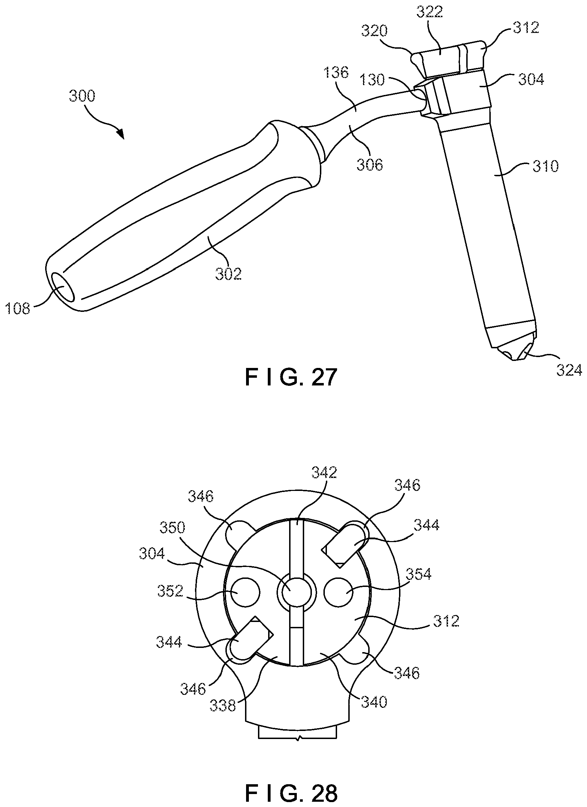

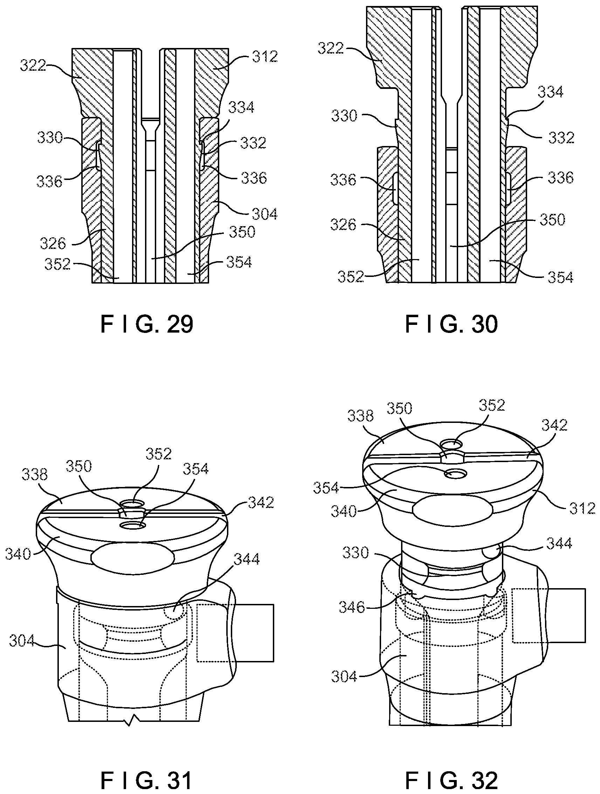

Figs. 27 - 37 depict a device 300 according to another example, not forming part of the invention. The device 300 is substantially similar to the device 100 except as pointed out below, with like elements referenced with like reference numerals. The device 300 comprises a handle 302 operably connected to a housing 304 via an extension 306. Similar to the extension 106, the extension 306 includes a tapered portion 136 extending out of the handle 302, the tapered portion 136 angled so that when the handle 302 is positioned at a desired angle relative to the bone (not shown), the housing 304 attached to the second end 130 lies in a plane substantially orthogonal to a longitudinal axis of the bone, as will be described in greater detail later on. It is noted, however, that the extension 306 may extend from the handle 302 at any other angle. The housing 304 is formed substantially similarly to the housing 104 and is operably connected to a protection sleeve 310 formed substantially similarly to the protection sleeve 110. Like the protection sleeve 110, the protection sleeve 310 is also integrally formed with (e.g., welded to) the housing 304.

A drill sleeve 312 is insertable into an opening 308 extending through the housing 304 and the protection sleeve 310. The drill sleeve 312 is formed substantially similar to the drill sleeve 116 and extends from a proximal end 320 comprising a head 322 to a distal end 324 along a shaft 326. A longitudinal axis of the drill sleeve 312 is aligned with a longitudinal axis of the protection sleeve 310. An outer diameter of the head 322 is greater than a diameter of the opening 308 of the housing to prevent axial movement of the head 322 distally into the housing 304. Axial movement of the drill sleeve 312 relative to the housing 304 is limited by a first axial movement limiting mechanism, as described herein. Specifically, the drill sleeve 312 comprises one or more radial abutments 330 distributed over an outer wall thereof distally of the head 322. The radial abutments 330 may comprise a first angled wall 332 and a second wall 334 extending substantially perpendicular to the longitudinal axis of the drill sleeve 312. As those skilled in the art will understand, this configuration permits the slidable insertion of the first angled wall 332 past the opening 308 and into the housing 304 to lockingly engage a radial groove 336 extending into an inner wall thereof. Specifically, once the radial abutments 330 have been slidably received within the radial groove 336, engagement of the second wall 334 with a proximal end of the radial groove 336 prevents withdrawal of the drill sleeve 312 from the housing. To withdraw the drill sleeve 312 from the housing 304, a user manually constricts first and second partitions 338, 340 of the head 377 defined by a cross-slot 342. Specifically, constriction of the first and second partitions 338, 340 causes a constriction of a proximal region of the drill sleeve 312, thereby releasing the radial abutments 330 from the radial groove 336. The radial abutments 330 may be provided on opposing locations of the drill sleeve 312 corresponding to the positions of the first and second partitions 338, 340.

Rotational movement of the drill sleeve 312 relative to the housing 304 may be controlled by a separate rotation control mechanism. Specifically, the drill sleeve may further comprise one or more buttons 344 formed on an outer wall thereof adjacent to a distal end of the head 322. In an exemplary embodiment, the button 344 is hemispherical, although other shapes are envisioned within the scope of the invention. In an exemplary embodiment, two buttons 344 may be provided on opposing walls of the drill sleeve 312, as shown in Fig. 28. The opening 308 may include a plurality of correspondingly shaped recesses 346 extending thereinto to permit insertion of the button thereinto. In an exemplary embodiment, the opening 308 may include four recesses 346 distributed thereover and separated from one another by approximately 90 degrees. In an operative configuration, a user may select a desired rotational orientation of the drill sleeve 312 relative to the housing 304 and insert the buttons 344 distally into the desired recesses 346 to lock the drill sleeve 312 in the desired orientation. Distal movement of the buttons 344 into the recesses 346 also causes the radial abutments 330 to engage the groove 336, thus permit a simultaneous locking of the drill sleeve 312 against axial movement and rotation relative to the housing 304.

A distal portion of the shaft 322 may be provided with a taper formed substantially similarly to the tapered portion of shaft 178 and configured to aid in insertion thereof to the target location in the body, as those skilled in the art will understand.

The drill sleeve 312 comprises first, second and third elongated channels 350, 352, 354 extending longitudinally therethrough and open to the proximal and distal ends 320, 324. The first channel 350 extends through a central longitudinal axis of the drill sleeve 312. A width of the cross-slot 342 is smaller than a diameter of the first opening 350 to aid in proper positioning of a guide wire therethrough, as will be described in greater detail below with respect to an exemplary method. The second and third channels 352, 354 are equidistant from the first channel 350 on opposing lateral sides thereof. It is noted, however, that the second and third channels 352, 354 may be provided in any configuration through the drill sleeve 312. Additionally, any number of additional channels may be used. The placement of the first, second and third channels 350, 352, 354 permits a user to insert multiple guide wires into the bone without removing the drill sleeve 312 or removing an earlier placed guide wire, as described in greater detail with respect to the exemplary method below.

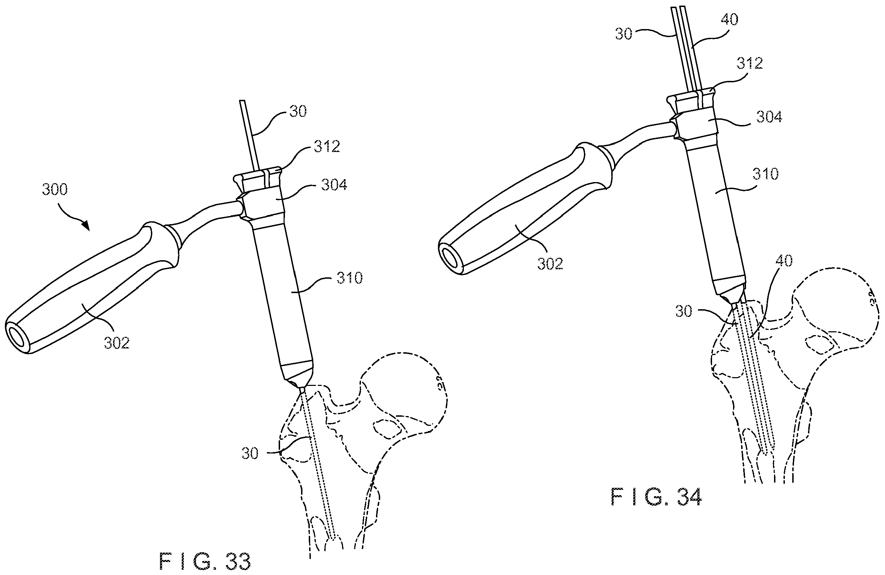

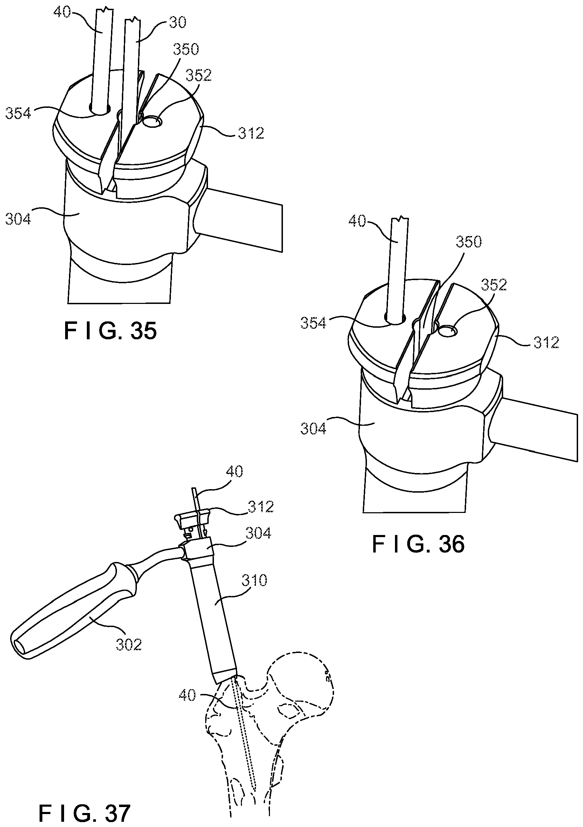

In accordance with an exemplary method, the device 300 is inserted into the body in an insertion configuration with the drill sleeve 312 locked in a first orientation relative to the housing 304, as shown in Fig. 27. In this position, the drill sleeve 312 is held within the protection sleeve 3 10 and housing 304 via a friction fit and locked against axial and rotation movement using the mechanisms described earlier. Once the distal end 324 of the drill sleeve 312 has made contact with an outer surface of a target bone (e.g., a proximal portion of the femur), a physician may optionally adjust the drill sleeve 312 to a desired orientation relative to the bone. Once the drill sleeve 312 is locked relative to the protection sleeve 310 and housing 304, a first guide wire 30 may then be inserted into the first channel 350, as shown in Fig. 33. If the physician determines that a position of the guide wire 30 is not accurate, a second guide wire 40 may be inserted through one of the second and third channels 352, 354 without removing the first guide wire 30. Specifically, after insertion of the first guide wire 30, the physician may determine that the drill sleeve 312 requires rotation relative to the housing 304. To do so, the first guide wire 30 may remain in place while a constrictive force is applied to the first and second partitions 338, 340, followed by a proximal withdrawal of the drill sleeve 312 by a distance sufficient to separate the radial abutment 330 from the head 322. The drill sleeve 312 may then be rotated as needed and moved distally within the housing 304 to once again lock a position thereof. The physician may then insert the second guide wire 40 through, for example, the third channel 354, as shown in Figs. 34 and 35. If the position of the second guide wire 40 is determined to be correct, the first guide wire 30 may be removed, as shown in Fig. 36. The drill sleeve 312 may then be removed from the housing 304 while leaving the guide wire 40 in place, as shown in Fig. 37. As those skilled in the art will understand, this permits the insertion of other devices (e.g., reamer, drill, etc.) through the protection sleeve 310 to complete the designated procedure.

It will be apparent to those skilled in the art that various modifications and variations may be made in the structure of the present invention, without departing from the scope of the invention. Thus, it is intended that the present invention cover the modifications and variations of this invention provided that they come within the scope of the appended claims.

## Claims

1. An aiming device (100; 200) for bone fixation, comprising:
a handle (102; 202) extending from a first end (120) to a second end (122) along a longitudinal axis;
a first guide arm (104, 106; 204, 206) connected to the second end (122) of the handle (102) and connectable to a protection sleeve (110);
a second guide arm (112; 212) connected to the first guide arm (104, 106; 204, 206);
a drill guide (116) configured and dimensioned for insertion through the protection sleeve (110), the drill guide (116) having a first guide channel (190) extending therethrough to guide an insertion of a guide wire into a bone, wherein the second guide arm (112; 212) is a blocking element and is movable with respect to the first guide arm (104, 106; 204, 206) between a first configuration wherein the drill guide (116) is axially movable relative to the second guide arm (112; 212) to a second configuration wherein the drill guide (116) is not axially movable relative to the second guide arm (112; 212),
**characterized in that** the first guide arm (104, 106; 204, 206) has a first opening (108) extending therethrough configured and dimensioned to receive the protection sleeve (110) therein, **in that** the second guide arm (112; 212) has a second opening (114) extending therethrough and being alignable with the first opening (108), and **in that** the drill guide (116) is configured and dimensioned for insertion through the second opening (114).

2. The aiming device of claim 1, wherein the second guide arm (112; 212) comprises a lever (150, 152; 213) actuatable by a user to move the second guide arm (112; 212) between the first and second configurations, the lever (150, 152; 213) configured to move the second guide arm (112; 212) relative to the first guide arm (104, 106; 204, 206) so that, in the first configuration, a central hole axis of the first opening (108) is longitudinally aligned with a central hole axis of the second opening (114) and, in the second configuration, the central hole axis of the first opening (108) is laterally offset from the central hole axis of the second opening (114).

3. The aiming device of claim 2, wherein the lever (150, 152) is configured to slide along a plane extending orthogonally to a longitudinal axis of the drill guide (116) to move the second guide arm (112; 212) between the first and second configurations.

4. The aiming device of claim 2, wherein the lever (213) is configured to pivot relative to the drill guide to move the second guide arm (112; 212) between the first and second configurations.

5. The aiming device of any one of claims 1 to 4, wherein, in the first configuration of the second guide arm (112; 212), the drill guide (116) is rotatable relative to the protection sleeve (110).

6. The aiming device of claim 5, wherein, in the second configuration of the second guide arm (112; 212), the drill guide (116) is one of rotatable and not rotatable relative to the protection sleeve (110).

7. The aiming device of any one of claims 1 to 6, further comprising a protection sleeve (110) extending from a proximal end (164) to a distal end (166), the proximal end (164) being configured to lockingly engage the first guide arm (104, 106), the distal end (166) being configured to engage a target portion of the bone in an operative configuration.

8. The aiming device of claim 7, wherein the distal end (166) of the protection sleeve (110) is angled to conform to a shape of the bone, the distal end (166) further comprising a pointed tip configured to frictionally engage the bone.

9. The aiming device of any one of claims 1 to 8, where the drill guide (116) comprises a second guide channel (190) axially offset from the first guide channel (190) and configured to receive the guide wire therethrough.

10. The aiming device of claim 3, wherein the first guide arm (104, 106) comprises a slotted portion (148) configured to slidably receive the second guide arm (112) therein.

11. The aiming device of any one of claims 1 to 10, wherein the second guide arm (112) comprises an abutment (158) extending into the second opening (114), the abutment (158) being configured to apply a frictional pressure on the drill guide (116).

12. The aiming device of any one of claims 1 to 11, wherein a proximal portion (184) of the drill guide (116) is threaded.

## Patentansprüche

1. Zielvorrichtung (100, 200) zur Knochenfixierung, umfassend:
einen Handgriff (102; 202), der sich von einem ersten Ende (120) zu einem zweiten Ende (122) entlang einer Längsachse erstreckt;
einen ersten Führungsarm (104, 106; 204, 206), der mit dem zweiten Ende (122) des Handgriffs (102) verbunden und mit einer Schutzhülse (110) verbindbar ist;
einen zweiten Führungsarm (112; 212), der mit dem ersten Führungsarm (104, 106; 204, 206) verbunden ist;
einen Bohrführung (116), konfiguriert und bemessen zum Einführen durch die Schutzhülse (110), wobei die Bohrführung (116) einen ersten, sich durch sie hindurch erstreckenden Kanal (190) aufweist zum Führen eines Einführvorgangs eines Führungsdrahts in einen Knochen, wobei der zweite Führungsarm (112; 212) ein Sperrelement ist und bewegbar ist in Bezug auf den ersten Führungsarm (104, 106; 204, 206) zwischen einer ersten Konfiguration, in welcher die Bohrführung (116) axial in Bezug auf den zweiten Führungsarm (112; 212) bewegbar ist, in eine zweite Konfiguration, in welcher die Bohrführung (116) nicht axial in Bezug auf den zweiten Führungsarm (112; 212) bewegbar ist,
**dadurch gekennzeichnet, dass** der erste Führungsarm (104, 106; 204, 206) eine sich durch ihn hindurch erstreckende erste Öffnung (108) aufweist, konfiguriert und bemessen zum Aufnehmen der Schutzhülse (110) in sich, dass der zweite Führungsarm (112; 212) eine zweite, sich durch ihn hindurch erstreckende zweite Öffnung (114) aufweist, die mit der ersten Öffnung (108) ausrichtbar ist, und dass die Bohrführung (116) konfiguriert und bemessen ist für das Einführen durch die zweite Öffnung (114).

2. Zielvorrichtung nach Anspruch 1, bei der der zweite Führungsarm (112; 212) einen Hebel (150, 152; 213) aufweist, der von einem Benutzer betätigbar ist, um den zweiten Führungsarm (112; 212) zwischen der ersten und der zweiten Konfiguration zu bewegen, wobei der Hebel (150, 152; 213) konfiguriert ist zum Bewegen des zweiten Führungsarms (112; 212) in Bezug auf den ersten Führungsarm (104, 106; 204, 206) derart, dass in der ersten Konfiguration eine zentrale Hohlachse der ersten Öffnung (108) in Längsrichtung ausgerichtet ist mit einer zentralen Hohlachse der zweiten Öffnung (114), und dass in der zweiten Konfiguration die zentrale Hohlachse der ersten Öffnung (108) seitlich gegenüber der zentralen Hohlachse der zweiten Öffnung (114) versetzt ist.

3. Zielvorrichtung nach Anspruch 2, bei der der Hebel (150, 152) konfiguriert ist zum Gleiten entlang einer Ebene, die sich orthogonal zu einer Längsachse der Bohrführung (116) erstreckt, um den zweiten Führungsarm (112; 212) zwischen der ersten und der zweiten Konfiguration zu bewegen.

4. Zielvorrichtung nach Anspruch 2, bei der der Hebel (213) konfiguriert ist zum Verschwenken relativ zu der Bohrführung, um den zweiten Führungsarm (112; 212) zwischen der ersten und der zweiten Konfiguration zu bewegen.

5. Zielvorrichtung nach einem der Ansprüche 1 bis 4, bei der in der ersten Konfiguration des zweiten Führungsarms (112; 212) die Bohrführung (116) in Bezug auf die Schutzhülse (110) drehbar ist.

6. Zielvorrichtung nach Anspruch 5, bei der in der zweiten Konfiguration der zweite Führungsarm (112; 212), die Bohrführung (116) entweder drehbar oder nicht-drehbar in Bezug auf die Schutzhülse (110) ist.

7. Zielvorrichtung nach einem der Ansprüche 1 bis 6, weiterhin umfassend eine Schutzhülse (110), die sich von einem proximalen Ende (164) zu einem distalen Ende (166) erstreckt, wobei das proximale Ende (164) konfiguriert ist zum verriegelnden Eingriff mit dem ersten Führungsarm (104, 106), während das distale Ende (166) konfiguriert ist für den Eingriff mit einem Zielbereich des Knochens in einer operativen Konfiguration.

8. Zielvorrichtung nach Anspruch 7, bei der das distale Ende (166) der Schutzhülse (110) abgewinkelt ist zur Anpassung an eine Form des Knochens, und das distale Ende (166) weiterhin eine zulaufende Spitze aufweist, konfiguriert für den Reibeingriff mit dem Knochen.

9. Zielvorrichtung nach einem der Ansprüche 1 bis 8, bei der die Bohrführung (116) einen zweiten Führungskanal (190) aufweist, der axial gegenüber dem ersten Führungskanal (190) versetzt ist und konfiguriert ist zum Aufnehmen des Führungsdrahts durch ihn hindurch.

10. Zielvorrichtung nach Anspruch 3, bei der der erste Führungsarm (104, 106) einen geschlitzten Abschnitt (148) aufweist, konfiguriert zum gleitenden Aufnehmen des zweiten Führungsarms (112) in sich.

11. Zielvorrichtung nach einem der Ansprüche 1 bis 10, bei der der zweite Führungsarm (112) einen Anschlag (158) aufweist, der sich in die zweite Öffnung (114) hinein erstreckt, wobei der Anschlag (158) konfiguriert ist zum Aufbringen eines Reibungsdrucks auf die Bohrführung (116).

12. Zielvorrichtung nach einem der Ansprüche 1 bis 11, bei der ein proximaler Abschnitt (184) der Bohrführung (116) mit Gewinde versehen ist.

## Revendications

1. Dispositif de visée (100 ; 200) pour la fixation osseuse, comprenant :
un manche (102 ; 202) qui s'étend depuis une première extrémité (120) jusqu'à une seconde extrémité (122) suivant un axe longitudinal ;
un premier bras de guidage (104, 106 ; 204, 206) qui est connecté à la seconde extrémité (122) du manche (102) et qui peut être connecté à un manchon de protection (110) ;
un second bras de guidage (112 ; 212) qui est connecté au premier bras de guidage (104, 106 ; 204, 206) ;
un guide de foret (116) qui est configuré et dimensionné dans le but de son insertion au travers du manchon de protection (110), le guide de foret (116) comportant un premier canal de guidage (190) qui s'étend au travers de lui-même pour guider une insertion d'un fil de guidage à l'intérieur d'un os, dans lequel le second bras de guidage (112 ; 212) est un élément de blocage et il peut être déplacé par rapport au premier bras de guidage (104, 106 ; 204, 206) entre une première configuration dans laquelle le guide de foret (116) peut être déplacé axialement par rapport au second bras de guidage (112 ; 212) et une seconde configuration dans laquelle le guide de foret (116) ne peut pas être déplacé axialement par rapport au second bras de guidage (112 ; 212) ;
**caractérisé en ce que** le premier bras de guidage (104, 106 ; 204, 206) comporte une première ouverture (108) qui s'étend au travers de lui-même et qui est configurée et dimensionnée pour recevoir en son sein le manchon de protection (110), **en ce que** le second bras de guidage (112 ; 212) comporte une seconde ouverture (114) qui s'étend au travers de lui-même et qui peut être alignée avec la première ouverture (108), et **en ce que** le guide de foret (116) est configuré et dimensionné dans le but de son insertion au travers de la seconde ouverture (114).

2. Dispositif de visée selon la revendication 1, dans lequel le second bras de guidage (112 ; 212) comprend un levier (150, 152 ; 213) qui peut être actionné par un utilisateur pour déplacer le second bras de guidage (112 ; 212) entre les première et seconde configurations, le levier (150, 152 ; 213) étant configuré pour déplacer le second bras de guidage (112 ; 212) par rapport au premier bras de guidage (104, 106 ; 204, 206) de telle sorte que, dans la première configuration, un axe de trou central de la première ouverture (108) soit aligné longitudinalement avec un axe de trou central de la seconde ouverture (114) et que, dans la seconde configuration, l'axe de trou central de la première ouverture (108) soit décalé latéralement par rapport à l'axe de trou central de la seconde ouverture (114).

3. Dispositif de visée selon la revendication 2, dans lequel le levier (150, 152) est configuré de manière à ce qu'il coulisse le long d'un plan qui s'étend perpendiculairement à un axe longitudinal du guide de foret (116) afin de déplacer le second bras de guidage (112 ; 212) entre les première et seconde configurations.

4. Dispositif de visée selon la revendication 2, dans lequel le levier (213) est configuré de manière à ce qu'il pivote par rapport au guide de foret afin de déplacer le second bras de guidage (112 ; 212) entre les première et seconde configurations.

5. Dispositif de visée selon l'une quelconque des revendications 1 à 4, dans lequel, dans la première configuration du second bras de guidage (112 ; 212), le guide de foret (116) peut être entraîné en rotation par rapport au manchon de protection (110).

6. Dispositif de visée selon la revendication 5, dans lequel, dans la seconde configuration du second bras de guidage (112 ; 112), le guide de foret (116) peut soit être entraîné en rotation par rapport au manchon de protection (110), soit ne pas être entraîné en rotation par rapport à ce même manchon de protection.

7. Dispositif de visée selon l'une quelconque des revendications 1 à 6, comprenant en outre un manchon de protection (110) qui s'étend depuis une extrémité proximale (164) jusqu'à une extrémité distale (166), l'extrémité proximale (164) étant configurée pour engager par verrouillage le premier bras de guidage (104, 106), l'extrémité distale (166) étant configurée pour engager une partie cible de l'os selon une configuration opérationnelle.

8. Dispositif de visée selon la revendication 7, dans lequel l'extrémité distale (166) du manchon de protection (110) est angulée de manière à ce qu'elle épouse une forme de l'os, l'extrémité distale (166) comprenant en outre une extrémité terminale profilée qui est configurée pour engager l'os par frottement.

9. Dispositif de visée selon l'une quelconque des revendications 1 à 8, dans lequel le guide de foret (116) comprend un second canal de guidage (190) qui est décalé axialement par rapport au premier canal de guidage (190) et qui est configuré pour recevoir le fil de guidage au travers de lui-même.

10. Dispositif de visée selon la revendication 3, dans lequel le premier bras de guidage (104, 106) comprend une partie à fente(s) (148) qui est configurée pour recevoir en son sein de façon coulissante le second bras de guidage (112).

11. Dispositif de visée selon l'une quelconque des revendications 1 à 10, dans lequel le second bras de guidage (112) comprend une butée (158) qui s'étend à l'intérieur de la seconde ouverture (114), la butée (158) étant configurée pour appliquer une pression de frottement sur le guide de foret (116).

12. Dispositif de visée selon l'une quelconque des revendications 1 à 11, dans lequel une partie proximale (184) du guide de foret (116) est filetée.
